# EUROPEAN PATENT APPLICATION

(11) **EP 1 602 467 A1**
(43) Date of publication of application: **07.12.2005**
(21) Application number: 05007662.9
(22) Date of filing: 07.04.2005
(51) Int. Cl.: B29C 59/06, B29D 7/01, A61F 13/15

(54) **Formed film having a cellularly defined base pattern and visible design**

(30) Priority: 12.04.2004 US 561446 P
(71) Applicant: Tredegar Film Products Corporation, Richmond, VA 23225 (US)
(72) Inventor: Thomas, Paul E., Terre Haute IN 47802-5066 (US)
(74) Representative: Finck, Dieter

(57) **Abstract**

A formed film is provided having a base pattern and a visible design both incorporated therein. The base pattern is comprised of base cells arranged in an interconnected network of groups or clusters. The visible design is defined by a series of design cells arranged successively to outline a perimeter of a desired design. The base cells differ from the design cells either in size and/or shape sufficiently to be visibly distinct and contrasted with one another. Absorbent articles are also provided that incorporate the formed film, as well as methods and structures for manufacturing the formed film.

## Description

### BACKGROUND

The embodiments relate generally to formed films configured to be used in absorptive articles and more particular to a formed film having a base pattern and visible design separately defined at the cellular level. The embodiments further relate to articles that includes films of the foregoing nature, as well as methods and structures for manufacturing formed films.

Absorptive articles, such as diapers, feminine napkins, panty liners, incontinence inserts, breast pads, bed pads, ground covers for weed prevention, hygenic or household wipes, and the like, have been utilized that incorporate one or more layers of formed film. The formed film layers have been proposed in a variety of constructions.

The term "film" as used throughout shall refer to thermoplastic polymer webs made from any variety of processes, for example but not limited to thermoplastic polymer webs made from cast and blown extrusion processes. The term "formed film" as used throughout shall refer to films produced by any one of several forming methods. Some exemplary forming methods involve placing a formable web in contact with a forming cylinder having a pattern of perforations or indentations.

Extrusion processes may be used to create a roll of precursor film that is then formed through one of several processes, such as reheat vacuum formed film (RHVFF), direct melt vacuum formed film (DMVFF), hydrojet formed film (HFF), vacuum formed film (VFF) processes and the like. In the DMVFF, RHVFF and VFF processes, the film is fed across a forming cylinder while a pressure differential (e.g., vacuum) is applied. The pressure differential draws the film onto the forming cylinder thereby forming a pattern in or through the film corresponding to the pattern of the forming cylinder. In the HFF process, high pressure liquid jets are utilized to form the pattern in or through the film.

The patterns on the film are comprised of cells that are grouped into repeating arrangements with the groups of cells nested within one another. Cells of similar shape and size that interconnect in a network are generally referred to as a base pattern. Once a desired base pattern is formed on the film, the film may be combined with various layers of other materials to form a variety of articles.

Absorptive articles have been proposed that contain designs on the surface, where the design is intended to afford an appealing cosmetic appearance, such as to create an association with commonly known items that are fresh, feminine and pleasant, such as flowers. On baby diapers, for example, teddy bears, stars, moons and toys such as rattles and blocks are commonly printed on the back sheet or outer covering.

Heretofore, the designs have been placed on the top side of absorptive articles, visible prior to using the device, through embossing methods. An embossing method generally involves, utilizing heat and pressure, to set a specific design into the surface of a pad. Once the heated, pressurized pad receives the design, the pad is cooled. However, conventional embossing methods have experienced certain drawbacks and limitations.

A need remains for a formed film that includes both a base pattern and visible design. Needs also remain for articles incorporating such formed films and for methods and structures to produce such formed films.

### BRIEF SUMMARY OF THE EMBODIMENTS

A formed film is provided having a base pattern defined by base cells. The formed film also includes a visible design defined by a series of design cells arranged successively with one another along a contour defining a perimeter of the visible design. The design cells and base cells are positioned in an intervening manner with one another to incorporate the visible design into the base pattern.

An absorbent article is provided that includes a formed film having a base pattern defined by base cells. The formed film also includes a visible design defined by a series of design cells arranged successively with one another along a contour defining a perimeter of the visible design. The design cells and base cells are positioned in an intervening manner with one another to incorporate the visible design into the base pattern.

A forming screen is provided adapted to produce a formed film. The screen includes a mesh body containing a screen base pattern and a screen visible design incorporated within the screen. The screen base pattern is defined by base openings through the mesh body of the screen. The screen visible design is defined by a series of design openings through the mesh body of the screen. The design openings are arranged successively with one another along a contour defining a perimeter of the visible design. The design openings and base openings are positioned in an intervening manner with one another to incorporate the screen visible design into the screen base pattern.

### BRIEF DESCRIPTION OF THE DRAWINGS

Figure 1 illustrates a top plan view of a film segment formed in accordance with an embodiment.

Figure 2 illustrates an enlarged view of a section of the film segment of Figure 1.

Figure 3 illustrates exemplary geometric shapes for design cells.

Figure 4 illustrates a film segment formed in accordance with an alternative embodiment.

Figure 5 illustrates a film segment formed in accordance with an alternative embodiment.

Figure 6 illustrates a film segment formed in accordance with an alternative embodiment.

Figure 7 illustrates a film segment formed with in accordance with an alternative embodiment.

Figure 8 illustrates a cross sectional view taken through line 8-8 in Figure 1.

Figure 9 illustrates a cross sectional view taken along line 9-9 in Figure 1.

Figure 10 illustrates a cross sectional view taken along line 10-10 in Figure 1.

Figure 11 illustrates an isometric view of an absorbent article formed in accordance with an embodiment.

Figure 12 illustrates a cross sectional view taken along line 12-12 in Figure 11.

Figure 13 illustrates a screen formed in accordance with an embodiment.

Figure 14 illustrates an enlarged view of a section of the screen of Figure 13.

### DETAILED DESCRIPTION OF THE EMBODIMENTS

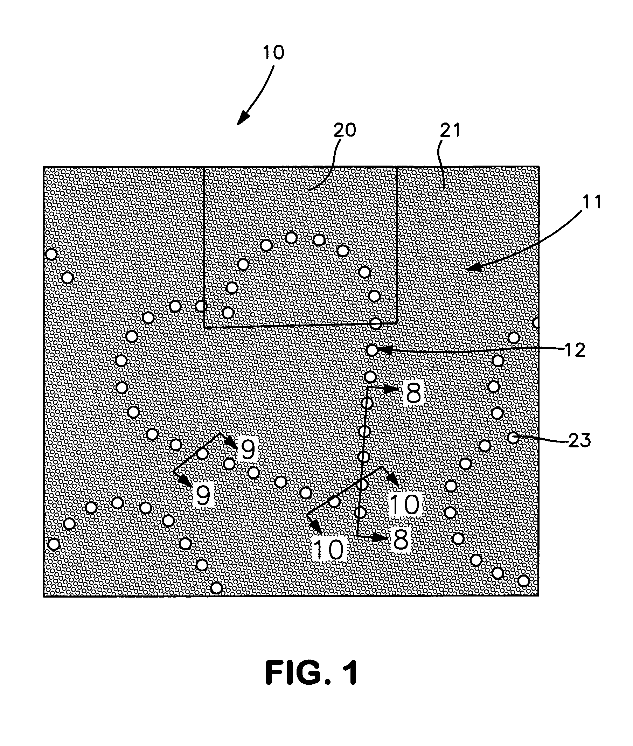

Figure 1 illustrates a film segment 10 formed in accordance with an embodiment. The film segment 10 generally comprises a base pattern 11 and visible design 12 integrated coplanar with one another. The base pattern 11 is defined by an interconnected network of base cells 21, while the visible design 12 is defined by a series of design cells 23. By way of example only, the visible design 12 draws a heart shape. In the example of Figure 1, the base cells 21 are circular depressions of a first uniform size and shape, while the design cells 23 are circular depressions of a different second uniform size and shape, wherein the base cells 21 are smaller than the design cells 23. The base and design cells 21 and 23 may have a variety of shapes, such as round, oval, elliptical, rectangular, square, triangular, stars, other polygonal shapes, boat shaped and the like. The base pattern 11 may also have a variety of shapes such as square, a hexagon, a round nesting of cells, cells forming nested pentagons, cells forming nested hexagons and the like.

The base and design cells 21 and 23 may constitute apertures extending entirely through the film segment 10 or may be non-perforating thereby only forming a depression into the film segment 10 with the valley of each depression being closed or covered by a membrane.

As used throughout, the term "cell" shall mean an individual depression having a geometric shape defined by the perimeter of the depression. Cells may open entirely through the film segment 10 or may be non-perforating or unapertured. When fluid acquisition is involved, the depression is in the direction away from the side of the material which will be exposed to the vision of the user and, while in use, contact the user. If a fluid barrier is involved the direction of the depression is optional.

As used throughout, the term "diameter" shall mean the lineal measurement of the major axis of a geometric shape of a cell. Whereas a circle, a square or an equilateral hexagon will have substantially equal measurements in both axes, other suitable geometric shapes shall have a major and a minor axis. The smaller cells may define the base pattern and the larger cells may define the visible design. The 'diameter' of significance is in the major axis measurement. The larger cells defining the perimeter of the visible design 12 may also be accentuated by being elongated, in which case the major axis shall determine the 'diameter'.

As used throughout, the term "base pattern" shall mean a group of one or more cells arranged in a repeating pattern where the groups of cells are nested in close proximity to one another to form an interconnected network. A film may have one or more base patterns. A base pattern may comprise cells of one or more size and/or geometric shape.

As used throughout, the term "visible design" shall mean a recognizable design defined by a series of similar cells. More than one size and shape of cells may be utilized to define the visible design. Examples of visible designs include hearts, roses, ribbons, flowers, toys, moons, stars and the like, or repeating patterns such as wavy lines, herringbone lines, chevrons and the like. The visible design has a perimeter set forth by a series of sequential aligned design cells. In the exemplary embodiments set forth hereafter, the interior area of the visible designs are wholly filled with base cells. Optionally, the interior area of some or all of the visible designs may be filled wholly or partially with design cells as well. Optionally, the interior area of some or all of the visible designs may be filled wholly or partially with filler cells having a size and/or geometric shape that differs from the base cells outside the visible design and that differs from the design cells arranged along the perimeter of the visible design.

The size and/or geometric shape the design cells is visibly distinct from the size and/or geometric shape of the base cells defining the base pattern. The perimeter of a visible design may be open such as in a wavy line, or it may be closed such as in a heart shape design. The visible design may be repeated at regular intervals to form a visible design pattern (e.g., a series of hearts arranged in rows or columns).

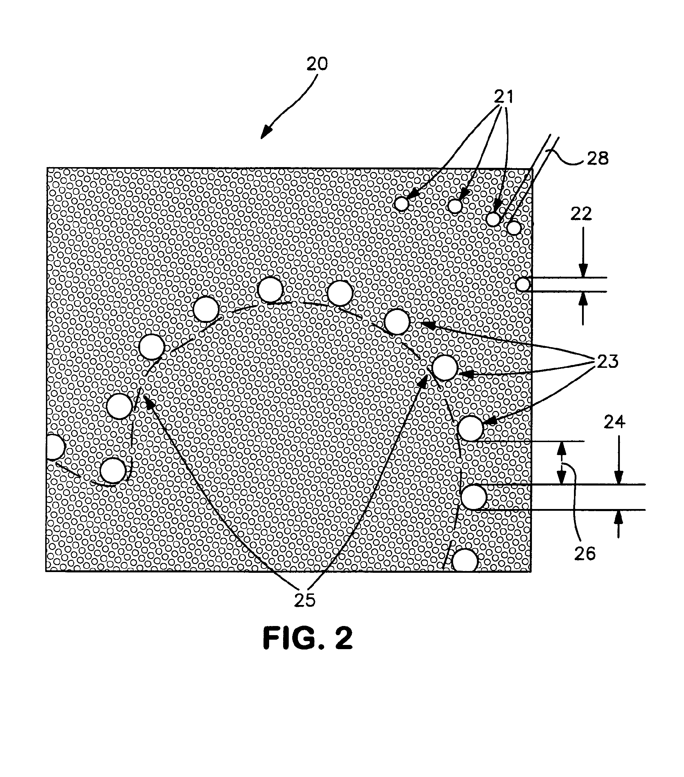

Figure 2 illustrates an enlargement of region 20 of the film segment 10 of Figure 1 to better illustrate the interrelation of the base cells 21 and design cells 23. The base pattern 11 comprises an interconnected network of base cells 21 having diameter 22. The visible design 12 comprises a series of larger design cells 23, all of which have substantially similar diameter 24. The design cells 23 are aligned in a sequential line forming perimeter 25 which defines the visible heart design. Adjacent design cells 23 in a common visible design 12 are spaced apart from one another by a design cell spacing 26. Adjacent base cells 21 in the base pattern 11 are spaced apart from one another by a common base cell spacing 28.

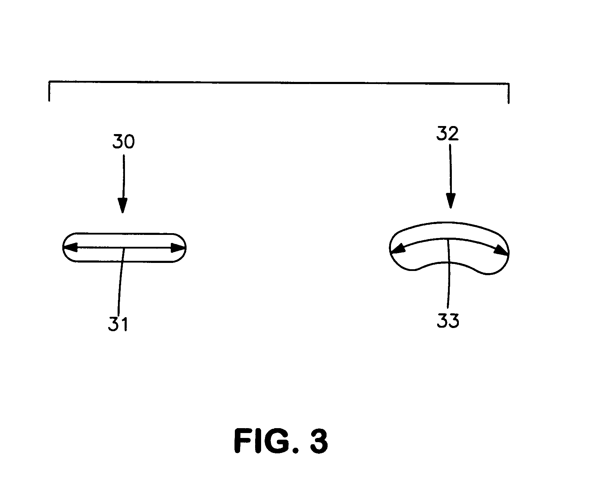

As shown in Figure 2, the base cell spacing 28 is less than the design cell spacing 26. In the base pattern 11 of Figure 2, each base cell 21 is surrounded by numerous (e.g., more than two) immediately neighboring base cells 21 of similar shape and size that are spaced uniformly from one another. In the visible design 12, each design cell 23 is bordered by a limited number (e.g., two) of opposed successive design cells 23. While each design cell 23 may be bordered by more than two design cells 23, the total number of bordering design cells 23 will be fewer than the number of base cells 21 that neighbor a representative base cell 21. Each design cell 23 is a discrete cell and is separate and distinct from the bordering design cells 23. Figure 3 illustrates elongated design cells 30 and 32 that are suitable, but not limiting, in accordance with one alternative embodiment. The elongated design cells 30 and 32 may be used to draw the visible design 12 onto a base pattern 11. By way of example, elongated design cell 30 may have substantially straight parallel sides with a linear major diameter 31, while the elongated design cell 32 may have curved sides which are substantially parallel with an arcuate major diameter 33.

The geometry of the base cells 21 used to form the base pattern 11 may or may not be the same. Preferably, diameters of the base cells 21 may be equivalent among the interconnected base cells 21 forming a cluster. Depending upon the difference in size between the base and design cells 21 and 23, it may be preferable that the geometries differ between the base and design cells 21 and 23. In the event that similar geometric shapes are used for the base and design cells 21 and 23, the size of design cells 23 should different sufficiently from the size of the base cells 21 to afford a desired contrast there between that is readily visible to a viewer at one to three feet.

By way of example only, a design cell 23 of a size (e.g., area or diameter) at least 2.00 to 3.00 times larger than the base cell 21 affords sufficient contrast to distinguish the base pattern 11 from the visible design 12. Preferably, the design cell 23 may have a diameter ranging anywhere between 2.25 and 2.50 times larger than that of the base cell 21, and most preferably the design cell 23 may have a diameter ranging anywhere between 2.00 and 2.25 times larger than the diameter of the base cell 21. Typically the design cell 23 size is at least about 2.00 times larger than the base cell 21 size, but may be greater than three times larger, such as four times or five times.

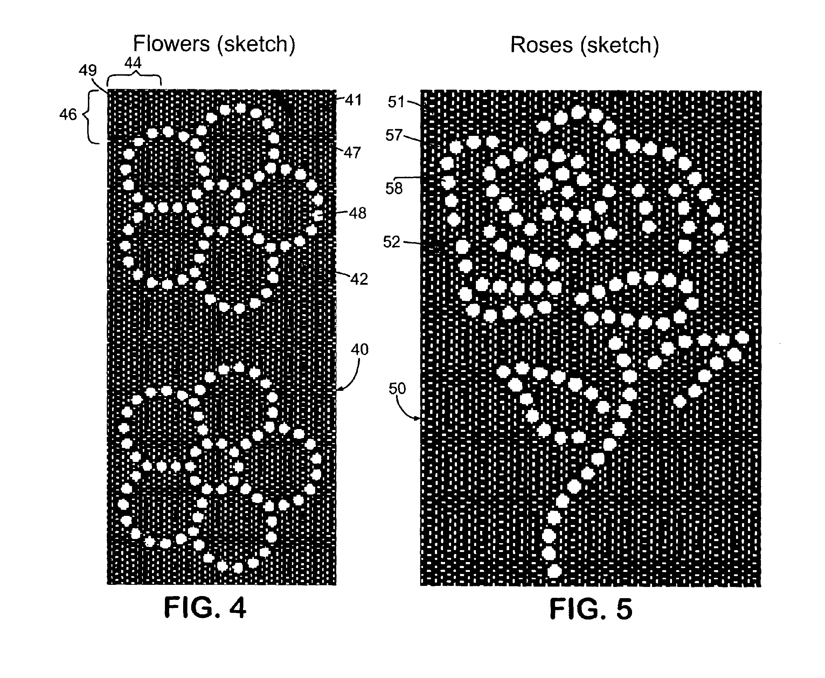

Figure 4 illustrates an alternative design for a film segment 40 comprised of a base pattern 41 and visible designs 42. The base pattern 41 is comprised of base cells 47, while the visible design 42 is comprised of design cells 48. The individual base cells 47 are configured with rectangular shapes, while the design cells 48 are circular. The base cells 47 are grouped in square clusters 49 generally arranged in a matrix of rows 46 and columns 44. Within each cluster 49 the base cells 47 are oriented in transverse patterns to one another. The visible design 42 represents a flower.

Figure 5 illustrates an alternative embodiment of a film segment 50 having a base pattern 51 and a visible design 52 formed thereon. The visible design 52 generally resembles a rose and is defined by a series of circular design cells 58. The base pattern 51 is defined by rectangular base cells 57.

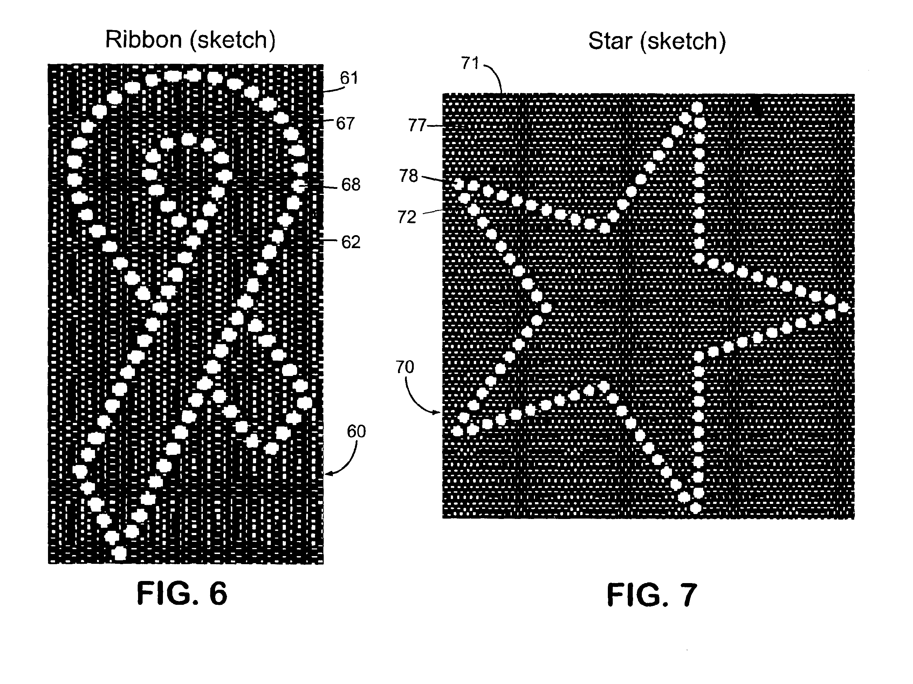

Figure 6 illustrates an alternative embodiment for a film segment 60 comprised of a base pattern 61 and a visible design 62. The visible design 62 is constructed to resemble a ribbon. The base pattern 61 is formed of rectangular base cells 67, while the visible design 62 is formed of circular design cells 68.

Figure 7 illustrates an alternative embodiment of a film segment 70 comprised of a base pattern 71 and a visible design 72. The base pattern 71 is comprised of rectangular base cells 77, while the visible design 72 is comprised of circular design cells 78 forming a star.

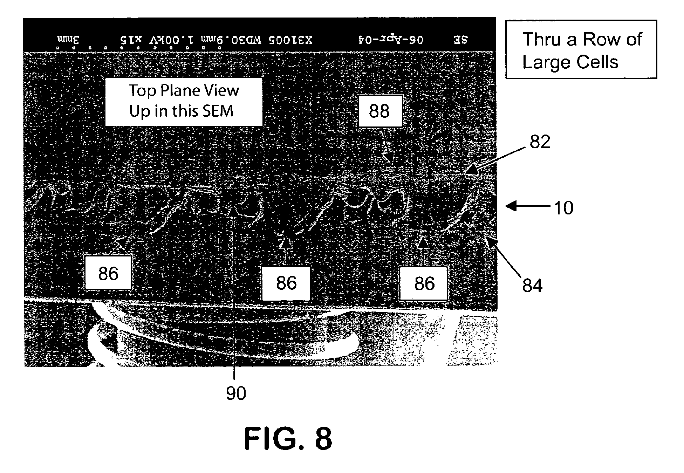

Figure 8 illustrates a side sectional view taken along line 8-8 in Figure 1 of the film segment 10. As shown in Figure 8, the film segment 10 includes a top or female surface 82 and a bottom or male surface 84. The top surface 82 is configured to face the user, while the bottom surface 84 is configured to be placed upon a forming cylinder (as explained below in more detail) when creating the base pattern 11 and visible design 12. The section line 8-8 is taken along a row of design cells 23 which are denoted by design impressions 86. The design impressions 86 are separated by linking segments 88. Each linking segment includes a smaller impression corresponding to a base impression 90.

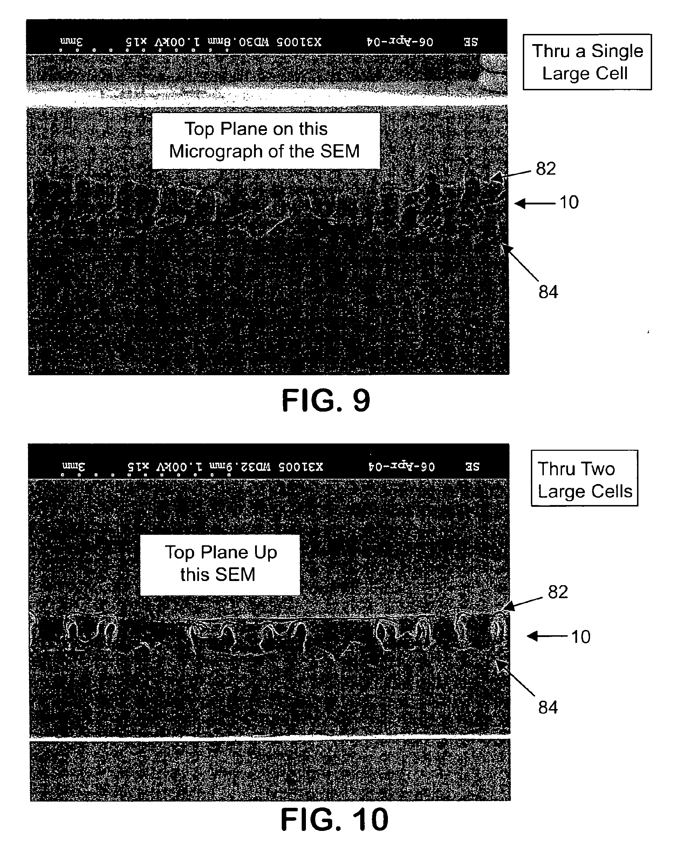

Figure 9 illustrates a cross sectional view taken along line 9-9 in Figure 1 through a single design cell 23 and multiple base cells 21 on either side of the design cell 23.

Figure 10 illustrates a cross sectional view taken along line 10-10 in Figure 1. The cross section illustrated in Figure 10 is taken through two design cells 23 which are separated by two base cells 21.

The film segment 10 may be utilized with a variety of articles.

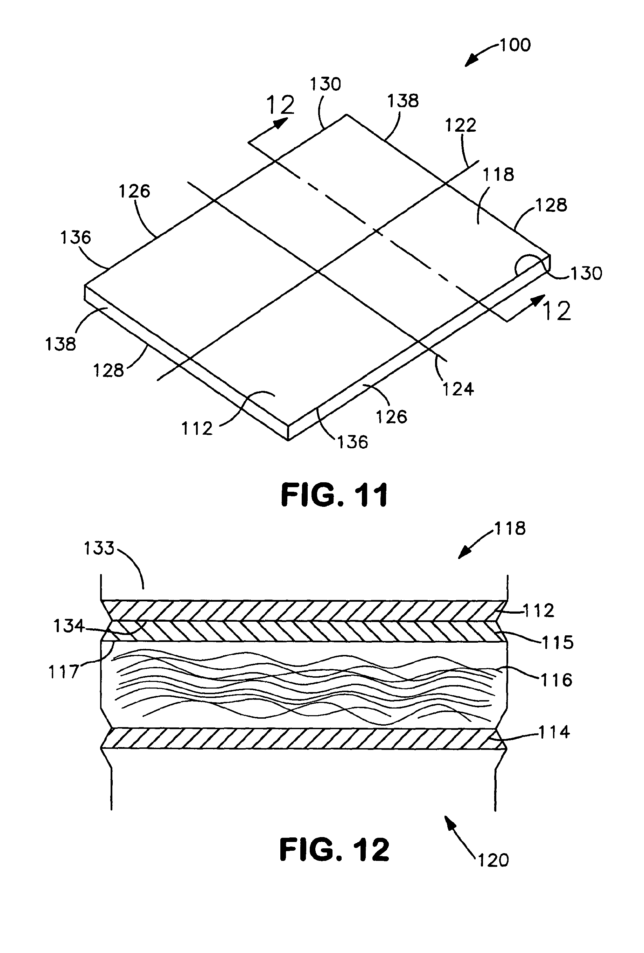

Figure 11 illustrates an exemplary article 100 within which the film segment 10 may be included. The article 100 is absorbent and comprises a top sheet 112, a back sheet 114, an acquisition distribution layer 115, and an absorbent core 116 (better shown in the cross sectional view of Figure 12).

The absorbent article 100 has two surfaces, namely a body contacting surface or body surface 118 and a garment contacting surface or garment surface 120. The body surface 118 is intended to be worn against the body of the wearer. The garment surface 120 of the absorbent article 100 is on the opposite side and is intended to be placed adjacent to the wearer's undergarments or clothing when the absorbent article 100 is worn.

The absorbent article 100 includes two center lines, a longitudinal center line 122 and a transverse center line 124. The absorbent article 100 has two spaced apart longitudinal edges 126 and two spaced apart transverse or end edges 128, which together form the periphery 130 of the absorbent article 100. The absorbent core 116 has a top or body facing side 117 (Fig. 12).

Top sheet 112 may be compliant, soft feeling and non-irritating to the wearer's skin. The top sheet 12 may be liquid permeable, permitting liquids to readily penetrate through its thickness. The top sheet has a body facing side 133 and a garment facing side 134, two longitudinal or side edges 136 and two end edges 138.

The top sheet 112 may be made of a non-woven material or a vacuum formed film layer, such as film segment 10 (Fig. 1). The top sheet 112 may be bonded to the acquisition distribution layer 115, although it need not be bonded, but instead may lay in contact with the acquisition distribution layer 115. The absorbent article 100 may utilize a three dimensional apertured plastic film as an anti-rewet or antiwicking layer. The three dimension apertured plastic film has a body facing side or female side and a garment facing side or male side. The garment facing side 134 of the top sheet 112 is preferably maintained in close contact with the female side of the apertured plastic film 44. The anti-rewet film may be in lieu of or in addition to the acquisition distribution layer 115.

The top sheet 112 may be any non-woven fabric that is permeable to liquids. A suitable non-woven fabric may be manufactured from a variety of materials including natural fibers (e.g. wool or cotton fibers), synthetic fibers (e.g. polyester, polypropylene) or a combination thereof. The top sheet 112 may be formed from fibers selected from the group consisting of polypropylene, polyester, polyethylene, polyvinylalcohol, starch based resins, polyurethanes, cellulose and cellulose esters.

Various manufacturing techniques may be used to manufacture non-woven fabric for use in the top sheet 112. For example, the non-woven fabric may be resin bonded, needle punched, spun-bonded, or carded. Carded non-woven fabrics may be thermally bonded, air-thru bonded and spun-laced fabrics. An exemplary non-woven fabric may be a thermally bonded polypropylene fabric.

An exemplary top sheet 112 is a non-woven fabric having a pattern of thermal bond sites. One example of a non-woven fabric has a carded thermally dot bonded polypropylene web. The thermal bonds of such a fabric are typically rectangular in shape in plan view. The bonds are typically arranged in staggered rows. Another typical non-woven is a spun-bonded polypropylene web with similarly arranged thermal bonds. Still another typical non-woven fabric is a carded polypropylene web that is embossed in accordance with the method taught in U.S. Pat. No. 4,781,710 issued to Megison, et al. This non-woven fabric has embossed and thermal bonded areas that are diamond-shaped in plan view. The diamond-shaped bonds are spaced apart and arranged in a diamond-shaped grid such as is shown in FIGS. 1 and 2 of the Megison, et al. patent. Typically, the embossing does not extend to the underlying core, however.

Preferably, acquisition distribution layer 115 is a perforated thermoplastic film with tapered capillaries which has a run off percent of less than about 10 percent and which has an increased liquid flow rate through the tapered capillaries. The method of making such a film includes a two-fold surface treatment, which is taught by U.S. Pat. Nos. 4,535,020 and 4,456,570 to Thomas et al. entitled, "Perforated Film" and "Treatment of Perforated Film", respectively. U.S. Pat. Nos. 4,535,020 and 4,456,570 are incorporated herein by reference. The method teaches that one surface treatment is provided by adding an internal chemical additive, namely a surfactant, to a film forming polyolefin resin. The additive is compounded or otherwise mixed or blended with the resin prior to the film being formed from the resin. After the film is formed the other surface treatment is accomplished by treating the film with a corona discharge treatment, which acts on the chemical additive to provide the perforated film with a zero or near zero percent run off.

The surfactant provides a film surface, which has greater polarizability than the polyolefin film would have without the surfactant being added. Higher surface polarity yields higher wettability. Although the chemically treated film is more polar than untreated film, corona discharge treatment of the film itself provides the desired maximum wettability. Any surfactant which achieves this polarity and which migrates to the surface of the film may be used in the embodiments.

Optionally, the article may be constructed with a layer in the area beneath the topsheet and on top of the core, which provides for both the acquisition and the distribution of fluids, especially secondary fluids that are introduced after initial fluids have previously been absorbed.

Optionally, the film segment discussed above may be used as the topsheet of the article. Here the aesthetically pleasing appearance of a formed film topsheet with visible designs can be well detected. Optionally, the film segments may also serve as part of the acquisition distribution layer. In order for the visible design to remain visible, the topsheet would be translucent. A topsheet may be, but is not limited to, a commonly used nonwoven. The denier and basis wieght and fiber diameter of the nonwoven is selected such that the topsheet is translucent to the layer beneath it. A layer of formed film may also be used as the topsheet in this construction. The pigmentation and gauge of the topsheet can be formulated to make the topsheet translucent to the layer underneath it. If desired a topsheet and an acquisition distribution layer comprised of the formed film such as in Figures 1-10 can be combined and conjoined in a single step process known as vacuum form lamination (VFL).

Optionally, the formed film may also be used as part of the backsheet barrier layer, in which case the formed film would be unapertured as a barrier layer.

Forming the large diameter cells in a drawn design can be achieved by any of several means. The most common means are those described above where RHVFF, DMVFF, VFL and HFF processes are discussed for the making of formed films. These processes may utilize a formed film pattern cylinder apparatus typically called a "screen". Screens can be made by several means. Electroplated screens are preferred but screens can be made by etching or laser cutting cylinders. Patterned lates can also be formed by these means which are then bent and welded into cylinders. Screens can be single ply or multiple plies. Screens can be designed to incorporate the base pattern and visible designs of Figures 1-10 by making the perforations in the screen comprise the base cell pattern of perforations and the design cell pattern of perforations. This is achieved by artwork where the artwork will mask the perforation in the case of electroplating and mask the material around the perforation in the case of etching. For laser cutting the artwork is typically loaded from a CAD drawing into a computer, which controls the laser's cutting pattern. Lasers may also be used to cut the masking materials for establishing the patterns for electroplating or ethching steps.

While making the formed film segments of Figures 1-10 by the VFF or HFF methods are some options, it is not limiting. Secondary operations can draw the design of cells of a virtually equivalent larger diameter onto the base pattern in order to create a visible design. Hot needle punching, perforating/embossing, hydrojet cutting, laser cutting or other means can be applied, as well.

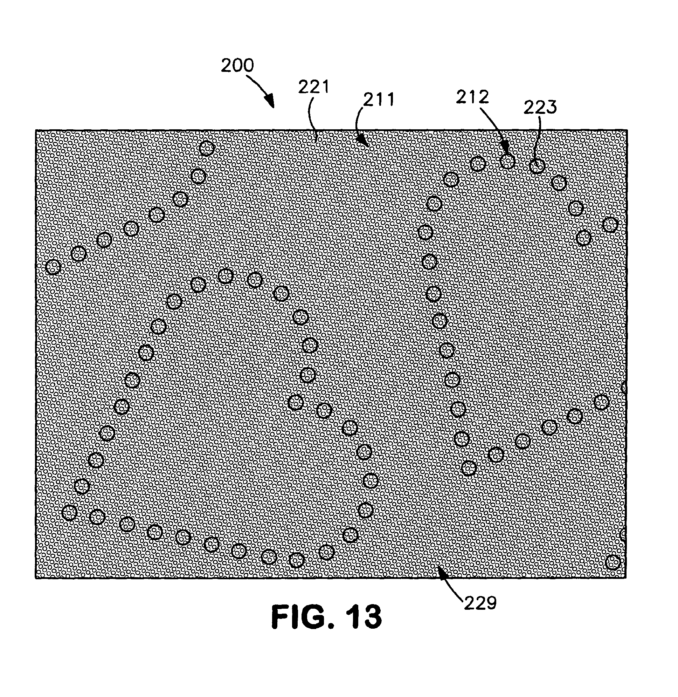

Figure 13 illustrates a screen 200 formed in accordance with an embodiment of the present invention. The screen 200 is comprised of a screen base pattern 211 and a screen visible design 212. The screen base pattern 211 is comprised of base openings 221 formed in groups defining clusters within an interconnecting network. The arrangement and relative description of the base openings 221 mirrors that described above in connection with Figure 1 for the base cells 21 and thus this discussion is not repeated here.

The screen visible design 212 is defined by a series of design openings 223 aligned in series and spaced from one another in a desired arrangement to outline the perimeter of the desired design. The relative orientation and relation of the design openings 223 mirrors that described above in connection with the design cells 23 illustrated in Figure 1 and thus that discussion is not repeated here.

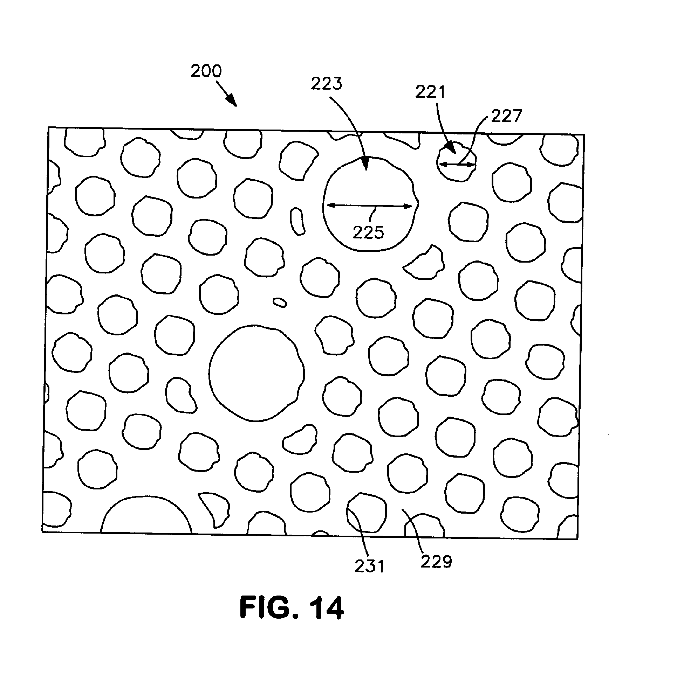

Figure 14 illustrates an exploded view of a portion of the screen 200 to better illustrate design openings 223 and base openings 221. The design openings 223 have a diameter 225, which is larger than the diameter 227 of base openings 221.

The screen 200 is comprised of a mesh body 229 formed of interconnecting links 231 surrounding the base and design openings 221 and 223.

The screen 200 is formed upon a cylinder or drum. A film web is introduced onto the screen 200 in a state susceptible to defamation. A pressure differential is introduced across the screen 200, such as having a vacuum drawn inward through the screen toward the central region of the cylinder. As the web is rolled over the screen 200, the pressure differential draws the web down onto the mesh body 229 and into the base and design openings 221 and 223. Should it be desired to form depressions in the web, a lesser pressure differential is utilized. In the event that it is desired to form apertures entirely through the web, a greater pressure differential is utilized.

Once the pattern is formed in the web and the web is removed from the screen 200, the resulting product constitutes a film segment such as illustrated above in connection with Figures 1-10.

The foregoing methods and structures form films having visible designs incorporated into a base pattern, in which the design cells and base cells are positioned in an intervening manner with one another.

In the exemplary embodiments, two levels of distinction are provided on the film, namely an array of visible designs (defining one level of distinction) and a base pattern (defining another level). Optionally, more than two levels may be incorporated into the film. To incorporate more than two levels, mid-level cells may be interleaved with the design cells and base cells. The mid-level cells would have a shape and/or size that is visibly distinct from the shape and/or size of the base cells and from the shape and/or size of the design cells.

While the various specific embodiments have been described herein, those skilled in the art will recognize that the invention can be practiced with modification within the spirit and scope of the claims.

## Claims

1. A formed film, said formed film comprising: a film containing a base pattern defined by base cells and a visible design defined by a series of design cells arranged successively with one another along a contour defining a perimeter of the visible design, the design cells and base cells being positioned in an intervening manner with one another to incorporate the visible design into the base pattern.

2. The formed file of claim 1 wherein the design cells differ from the base cells in at least one of size and geometric shape.

3. The formed file of claim 1 wherein the design cells differ from the base cells by an amount sufficient to visibly contrast with one another when the film is viewed from a distance of at least 12 inches.

4. The formed film of claim 1 wherein the film contains an array of visible designs, all of the visible designs having a common design contour.

5. The formed film of claim 1 wherein the film contains an array of visible designs, the visible designs including at least two different design contours.

6. The formed film of claim 1 wherein the base cells of the base pattern all have a common geometric shape.

7. The formed film of claim 1 wherein the design cells are larger than the base cells of the base pattern.

8. The formed film of claim 1 wherein the design cells are at least approximately 2.00 times larger in size than the base cells of the base pattern.

9. The formed film of claim 1 wherein the design and base cells all have a common geometric shape.

10. A method of forming a film, comprising:
providing a film;
defining a base pattern by arranging base cells in an interconnecting network;
defining a visible design by arranging a series of design cells along a contour forming a perimeter of the visible design; and
incorporating the visible design into the base pattern on the film by providing the design cells and base cells on the film in an intervening manner.

11. The method of claim 10 wherein the step of defining a visible design further comprises defining one of an open perimeter and a closed perimeter for the visible design.

12. The method of claim 10 further comprising arranging an array of the visible designs on the film where all of the visible designs in the array have a common design contour.

13. The method of claim 10 where the base cells of the base pattern all have a common geometric shape.

14. The method of claim 10 where the design cells defining the visible design are larger than the base cells of the base pattern.

15. The method of claim 10 where the design cells defining the visible design are at least approximately 2.00 times larger in size than the base cells of the base pattern.

16. The method of claim 10 where the design cells defining the visible design have the same geometric shape as the base cells of the base pattern.

17. The method of claim 17 where each design cell is spaced apart from and bordered by at least two design cells, and each base cell is surrounded by neighboring base cells

18. An absorptive article comprising:
an absorbent core configured to absorb fluid; and
a film layer containing a base pattern defined by base cells and a visible design defined by a series of design cells arranged successively with one another along a contour defining a perimeter of the visible design, the design cells and base cells being positioned in an intervening manner with one another to incorporate the visible design into the base pattern.

19. The absorptive article of claim 18 wherein said film layer constitutes at least a portion of topsheet layer configured to face a user.

20. The absorptive article of claim 18 further comprising a topsheet, wherein said film layer constitutes at least a portion of an acquisition distribution layer disposed beneath the topsheet and above the absorbent core.

21. The absorptive article of claim 18 further comprising a topsheet that is translucent to expose the visible design, the film layer being disposed between the absorbent core and the topsheet.

22. The absorptive article of claim 18 further comprising a topsheet formed of a non-woven material.

23. The absorptive article of claim 18 where each design cell is spaced apart from and bordered by at least two design cells, and each base cell is surrounded by neighboring base cells.

24. The absorptive article of claim 18 where the film layer constitutes at least a portion of a backsheet layer.

25. The absorptive article of claim 18 wherein the film layer is unapertured.

26. The absorptive article of claim 18 further comprising at least two film layers, the film layers comprising formed films.

27. The absorptive article of claim 18 further comprising at least two film layers arranged and constructed to constitute at least part of a topsheet and a backsheet with the absorbent core positioned therebetween.

28. The absorptive article of claim 18 further comprising at least two film layers arranged and constructed to constitute at least part of an acquisition distribution layer and a backsheet with the absorbent core positioned therebetween.

29. A screen for making formed films, said screen comprising: a mesh body containing a screen base pattern defined by base openings through the mesh body and a screen visible design defined by a series of design openings through the mesh body, the design openings being arranged successively with one another along a contour defining a perimeter of the screen visible design, the design openings and base openings being positioned in an intervening manner with one another to incorporate the screen visible design into the screen base pattern.

30. The screen of claim 29 wherein the design openings differ from the base openings in at least one of size and geometric shape.

31. The screen of claim 29 wherein the design openings differ from the base openings by an amount sufficient to visibly contrast with one another when the film is viewed from a distance of one and three feet.

32. The screen of claim 29 wherein the screen contains an array of screen visible designs, all of the screen visible designs having a common design contour.

33. The screen of claim 29 wherein the base openings of the screen base pattern all have a common geometric shape.

34. The screen of claim 29 wherein the design openings have one of a different geometric shape and a larger size than the base openings of the screen base pattern.
